# EUROPEAN PATENT SPECIFICATION

(11) **EP 1 279 730 B1**
(45) Date of publication and mention of the grant of the patent: **10.01.2007**
(21) Application number: 01118354.8
(22) Date of filing: 27.07.2001
(51) Int. Cl.: C12N 15/10, C12N 15/867, C12N 15/62, C12N 5/16, A01K 67/027

(54) **Methods for screening for proteins comprising a signal sequence**
Methode zum Auffinden von Proteinen mit einer Signalsequenz
Procédé de criblage de protéines comprenant une séquence de signal

(43) Date of publication of application: 29.01.2003
(73) Proprietor: FrankGen Biotechnologie AG, 61476 Kronberg/Taunus (DE)
(72) Inventor: Von Melchner, Harald, Prof. Dr, 61476 Kronberg/Taunus (DE); Gebauer, Mathias Lab. for Molecular Hematology, 60590 Frankfurt am Main (DE); Beckers, Thomas Lab. for Molecular Hematology, 60590 Frankfurt am Main (DE)
(74) Representative: Helbing, Jörg

(56) References cited:
- WO-A-00/09681
- DATABASE MEDLINE [Online] US NATIONAL LIBRARY OF MEDICINE (NLM), BETHESDA, MD, US; FELDHAUS A L ET AL: "A CD2 / CD28 chimeric receptor triggers the CD28 signaling pathway in CTLL.2 cells." retrieved from STN Database accession no. 97479360 XP002180072 & GENE THERAPY, (1997 AUG) 4 (8) 833-8. ,
- DATABASE CHEMICAL ABSTRACTS [Online] Pat. No. JP10313868: Database an 130:77954, 1998 XP002180073
- WILES MV ET AL.: "Establishment of a gene-trap sequence tag library to generate mutant mice from embryonic stem cells" NATURE GENETICS, vol. 24, January 2000 (2000-01), pages 13-14, XP002180070
- ANDREU T ET AL.: "Gene Trapping identifies inhibitors of oncogenic transformation" THE JOURNAL OF BIOLOGICAL CHEMISTRY, vol. 273, no. 22, 29 May 1998 (1998-05-29), pages 13848-13854, XP002180071

## Description

The present invention relates to plasmids or retroviral vectors comprising a human CD2 cell surface antigen fused in frame with a reporter gene and to methods of screening for a nucleic acid sequence encoding a protein comprising a signal sequence. In particular, methods are provided for screening for a nucleic acid sequence encoding a signal sequence protein that is regulated during a biological process.

### Background Of The Invention

Because defects in intracellular signaling can cause cancer, components of signal transduction pathways are common targets of antineoplastic drugs. Proteins targeted to the secretory pathway and cell-surface proteins have generated considerable interest for two main reasons. First, most fundamental biological processes involve secretory proteins and membrane receptors. Second, secreted and membrane-bound proteins are easily accessible to specific agonists or antagonists, such as exogenous drugs and are thus preferred targets for drug development. For example, many epithelial cancers show constitutive activation of the tyrosine kinase receptors for epidermal growth factor (EGF) or insulin-like growth factor (IGF) (Schlessinger, J. (2000) *Cell* 103, 211-25); this has led to the development of drugs that interfere with these receptors.

The targeting of proteins to the secretory pathway requires a short, aminoterminal sequence called a "signal" or leader" peptide (von Heijne, G. (1985) *J Mol Biol* **184,** 99-105) which also determines the protein's orientation across the cellular membranes. This signal sequence is conserved in secreted and membrane spanning proteins and has been exploited in all strategies developed to isolate or identify signal sequence genes. Thus, signal sequence traps (SST) consisting of reporter- or selectable marker genes, whose expression is dependent on the acquisition of a signal sequence, or antibodies raised against signal sequence peptides, have been used with variable success (Tashiro, K., et al. (1993) *Science* **261,** 600-3, Skarnes, W. et al. (1995) *Proc Natl Acad Sci USA* **92,** 6592-6, lmai, T., et al. (1996) *J Biol Chem* **271,** 21514-21, Klein, R. D., et al. (1996) *Proc Natl Acad Sci USA* **93,** 7108-13, Scherer, P. E. et al. (1998) *Nat Biotechnol* **16,** 581-6, Lim, S. P. & Garzino-Demo, A. (2000) *Biotechniques* **28**, 124-6, 128-30, Mitchell K.J. et al. (2001) Nat Genet 28, 241-9). To develop a strategy which would be applicable to genome-wide screens for secreted and transmembrane proteins in living cells, would be highly desirable.

Gene traps insert a reporter gene mostly into random chromosomal sites, including transcriptionally active regions. By selecting for gene expression, recombinants are obtained in which the reporter gene is fused to the regulatory elements of endogenous genes. Transcripts generated by these fusions faithfully reflect the activity of the tagged cellular gene and thus provide an effective means to study the expression of genes in their normal chromosomal location (Friedrich, G. & Soriano, P. (1991) *Genes Dev* **5,** 1513-1523, Skarnes, W. C., et al. (1992) *Genes Dev* **6,** 903-18, von Melchner, H., et al. (1992) *Genes Dev* **6,** 919-927). US 5,364,783 describes a promoter trap, wherein a retrovirus has a promoterless protein coding sequence located in the retroviral U3 or U5 region. By using appropriate reporter systems, it has been possible to identify genes that are either induced or repressed during important biological processes such as cell differentiation, programmed cell death or oncogenic transformation (Reddy, S., et al. (1992) *Proc Natl Acad Sci USA* **89,** 6721-6725, Russ, A. P., et al. (1996) *Proc Natl Acad Sci USA* **93,** 15279-15284, Forrester, L., et al. (1996) *Proc Natl Acad Sci USA* **93,** 1677-1682, Thorey, I. S., et al. (1998) *Mol Cell Biol* **18,** 3081-3088, Andreu, T., et al. , H. (1998) *J Biol Chem* **273,** 13848-54). Moreover, reporter genes dependent on signal sequences for expression have been used to directly screen the genome for secretory proteins. For example, a gene trap encoding a β-galactosidase/neomycin-phosphotransferase (βgeo) fusion protein into which a CD4 transmembrane domain had been inserted was shown to enrich for integrations into signal sequence genes expressed during mouse development (EP 0731169, Skarnes, W. C. et al. (1995) *Proc Natl Acad Sci USA* **92,** 6592-6, Mitchell K.J. et al. (2001) Nat Genet **28,** 241-9). However, since the efficiency of gene trap activation by signal sequence capture was less than 20%, this gene trap seems unlikely to be suitable for large scale functional genomics. WO 00/24881 describes a gene trap vector comprising a secretory trap module (type II transmembrane domain and a lumen-sensitive marker) and an axonal reporter to mark the axons of only those cells that normally express the trapped gene. With this method an average of 20 genuine secretory trap events per electroporation and plating of 300 colonies was achieved. Moreover, the method requires prescreening for "secretory" patterns by replica plating and lacZ-staining. This is laborious and time consuming.

On the other hand fusion constructs comprising CD2 and a sequence serving as a marker are known from A.C. Feldhaus, Gene Therapy 4(8) 833-838 (1997) and JP 1997-130952.

From the above-identified prior art, it is evident that there is a need for a method of screening for secreted proteins, as the prior art could not provide an efficient method, which can be carried out in large scale. In particular a method of screening for secreted proteins, which are regulated by a biological process would be highly desirable. Thus, a screening system that would effectively select for integrations into regulated genes encoding secreted and/or transmembrane proteins is needed.

A further object of the invention was to provide a high-throughput screening system and a method to screen for proteins with signal sequences. As a solution to this object a plasmid or a vector is provided wherein a CD2 cell surface antigen, preferably the human CD2 cell surface antigen, is fused in frame with a reporter gene and methods of screening for a nucleic acid sequence encoding a protein comprising a signal sequence. In particular, methods are provided for screening for a nucleic acid sequence encoding a signal sequence protein that is regulated in a biological process.

### Description Of The Invention

The present invention is directed to a plasmid or a retroviral vector comprising a fusion gene, wherein said fusion gene comprises a nucleic acid sequence encoding the cell surface antigen CD2, wherein said CD2 cell surface antigen has no translation start site and encodes at least the transmembrane and extracellular domains of the CD2 cell surface antigen, fused in frame to a nucleic acid sequence encoding a reporter gene.

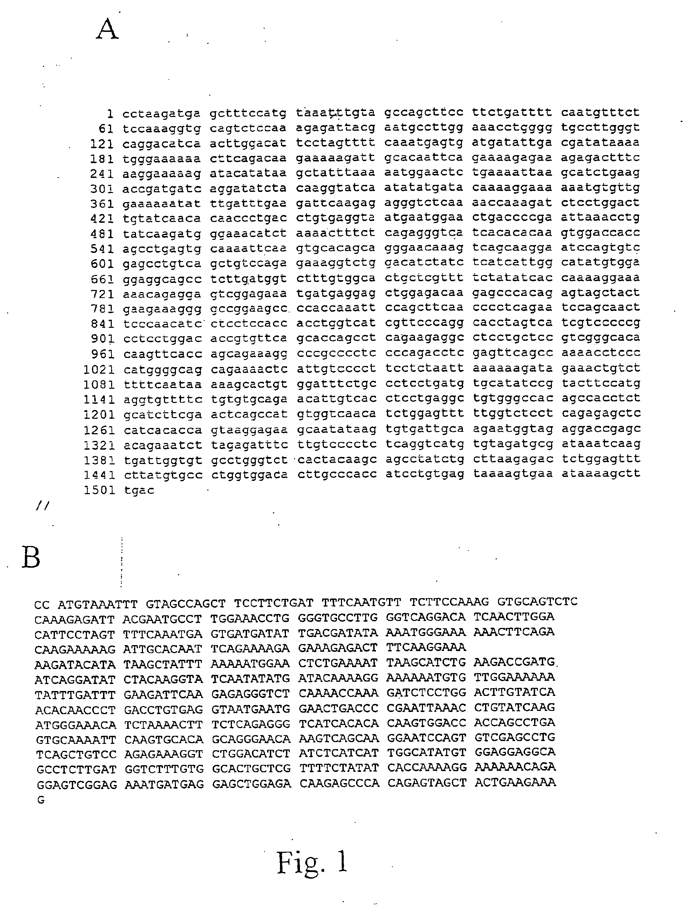

In a preferred embodiment the fusion gene comprises a nucleic acid sequence encoding a truncated CD2 cell surface antigen fused in frame to a nucleic acid sequence encoding a reporter gene. The nucleotide sequence of the human CD2 cell surface antigen cDNA (GenBank Acc# XM_002141) is shown in Figure 1A and SEQ ID NO:1. Preferably, the nucleic acid sequence encoding a truncated CD2 cell surface antigen is promoterless.

As det forth above, the nucleic acid sequence encoding a truncated CD2 cell surface antigen has no translation start site and encodes at least the extracellular and transmembane domains of the CD2 cell surface antigen. More preferably the nucleic acid sequence encoding a truncated CD2 cell surface antigen comprises nt. 10-79 after the translation start site (SEQ ID NO:2) encoding the extracellular domain of the human CD2 cell surface antigen (e.g. nucleotides 16-85 of the nucleotide sequence of the human CD2 cell surface antigen as shown in Figure 1A) and nt. 80-624 after the translation start site (SEQ ID NO:3) encoding the transmembrane domain of the human CD2 cell surface antigen (e.g. nucleotides 86-630 of the nucleotide sequence of the human CD2 cell surface antigen as shown in Figure 1A) of the CD2 cell surface antigen cDNA sequence. Most preferably, the nucleic acid sequence encoding a truncated CD2 cell surface antigen comprises the nucleotides 10-782 after the translation start site of the CD2 cDNA (e.g. nucleotides 16-788 of the nucleotide sequence of the human CD2 cell surface antigen of Figure 1A). The sequence consisting of the nucleotides 10-782 after the translation start site of the CD2 cDNA has been shown in Figure 1B and SEQ ID NO:4.

In a most preferred embodiment the fusion gene is inserted in the U3 or U5 region of a retroviral vector.

In a preferred embodiment the reporter gene is neomycin-phosphotransferase.

The above-specified plasmid or retroviral vector is suitable for the use in the method of present invention, which is directed to screening for a nucleic acid sequence encoding a protein comprising a signal sequence. Said method comprises the following steps: a) transfecting or infecting susceptible cells with the plasmid or vector according to the invention, b) selecting the cells wherein the plasmid or vector had integrated into the genome, wherein the CD2 signal sequence had been excised by splicing and wherein the integrated plasmid or vector is transcribed, c) detecting expression of CD2 or the fused reporter gene in the selected cells, wherein expression of CD2 or of the fused reporter gene is indicative of an integration of the plasmid or vector into a nucleic acid sequence encoding a protein comprising a signal sequence, and d) isolating said CD2-positive and/or fused reporter gene expressing cells. Since the signal peptide in the CD2 cDNA terminates in a cryptic splice acceptor site (Andreu, T. et al. (1998) *J Biol Chem* **273,** 13848-54), it is removed by splicing due to integrations into introns of expressed genes. Consequently, the fusion gene's activation relies on the acquisition of a signal sequence from an endogenous gene.

In a preferred embodiment the method of the present invention is adapted to screen for nucleic acid sequences which encode a protein comprising a signal sequence, wherein said protein is regulated during a biological process. Said adapted method further comprises the steps of: e) generating a CD2-positive library of CD2 expressing cells, f) treating the CD2-positive library with an agent, which initiates a biological process, g) analyzing the CD2 expression of the cells, wherein lack of CD2 expression is indicative of integration of the plasmid or vector into a nucleic acid sequence encoding a protein with a signal sequence regulated during a biological process, h) selecting the cells which do not express CD2, i) generating a CD2-negative library, j) withdrawing said agent, thereby terminating said biological process, k) analyzing the CD2 expression of the cells, wherein the induction of the CD2 expression is indicative of an integration of the plasmid or vector into nucleic acid sequence encoding a protein with a signal sequence, wherein said protein is regulated during the biological process, and I) isolating the CD2-positive cells.

In order to characterize the integrated sequences the method preferably further comprises the steps of: m) amplifying cellular sequences adjacent to the plasmid or provirus by genomic PCR and/or amplifying cell-plasmid/provirus fusion transcripts by RT-PCR, n) sequencing amplification products, and o) gene identification by data base searches with the sequence of the amplification products.

Transfecting or infecting susceptible cells with the plasmid or vector according to the invention denotes herein transducing said plasmid or vector into the cells.

In the present invention selecting the cells wherein the plasmid or vector had integrated into the genome, wherein the CD2 signal sequence had been excised by splicing and wherein the integrated plasmid or vector is transcribed refers to choosing the cells, wherein the plasmid or vector has integrated in their genome so that the CD2 signal sequence of the fusion gene has been deleted and the fusion gene is expressed. Selecting the cells can be preferably carried out by G418 selection.

Detecting expression of CD2 or of the fused reporter gene in the selected cells, wherein the expression of CD2 or of the fused reporter gene is indicative of an integration of the plasmid or vector into a nucleic acid sequence encoding a protein comprising a signal sequence, encompasses observing if CD2 or the fused reporter gene is transcribed and/or translated this showing that the plasmid or vector is integrated in the genome of said cells and that the plasmid or vector is fused with a nucleotide sequence encoding an endogenous signal peptide. Detecting the expression can be carried out with e.g. antibodies preferably directed against the CD2 antigen.

Isolating the CD2-positive and/or fused reporter gene expressing cells refers to separating the cells, which express CD2 and/or the fused reporter gene.

Generating a CD2-positive library of CD2 expressing cells denotes herein selecting, isolating and combining those cells which express CD2.

Treating the CD2-positive library with an agent which initiates a biological process encompasses addition of a composition to the cells which induces a change in the state of the cell, e.g. apoptosis, senescence or oncogenic transformation.

Analyzing the CD2 expression of the cells wherein lack of the CD2 expression is indicative of integration of the plasmid or vector into nucleic acid sequence encoding a protein with a signal sequence during a biological process, relates to observing if CD2 is transcribed and/or translated this showing that the plasmid or vector is integrated in the genome of said cells and that the plasmid or vector is fused with a nucleotide sequence encoding an endogenous signal sequence protein and this protein is controlled by an agent which induces a change in the state of the cell.

Selecting the cells which do not express CD2 denotes choosing the cells in which CD2 is not transcribed.

Generating a CD2-negative library encompasses selecting, isolating and combining those cells which do not express CD2.

Withdrawing the agent which initiates a biological process thereby terminating the process relates to eliminating the composition which induces the change of the state from the cells this reversing the state of the cells to the original, which means the state of the cells before treating with the agent.

Analyzing the CD2 expression of the cells, wherein the induction of CD2 expression is indicative of an integration of the plasmid or vector into nucleic acid sequence encoding a protein with a signal sequence wherein said protein is regulated during a biological process relates to observing if CD2 is transcribed and/or translated this showing that the plasmid or vector is integrated in the genome of said cells and that the plasmid or vector is fused with a nucleotide sequence encoding an endogenous signal peptide comprising protein and that this protein is controlled by an agent which induces a change in the state of the cell.

Isolating the CD2-positive cells refers to separating the cells, which express CD2.

Amplifying cellular sequences adjacent to the plasmid or provirus by genomic PCR and/or amplifying cell-provirus fusion transcripts by RT-PCR denotes multiplying endogenous nucleotide sequences in the vicinity of (next to) the plasmid or vector by polymerase chain reaction (an amplification technique using multiple cycles of polymerization, each followed by a brief heat treatment to separate complementary strands) or multiplying the endogenous signal sequence-plasmid/vector fusion transcripts by RT-PCR (a two step protocol for synthesizing cDNA molecules, wherein cDNA strands are synthesized by reverse transcriptase, mRNA as a template, and the specific cDNA strand is amplified by PCR).

Sequencing amplification products relates to determining the nucleotide composition of the products obtained by PCR.

Gene identification by data base searches with the sequence of the amplification products denotes detection of genes using computer nucleotide or protein homology search programs and the sequences of the amplification products.

In a preferred embodiment of the invention the signal sequence is a signal sequence of a secreted or of a transmembrane protein.

In another preferred embodiment the biological process of the method of the present invention is oncogenic transformation, cell differentiation, senescence, apoptosis or drug susceptibility. Oncogenic transformation as used herein is directed to a conversion of cells to a state of unrestrained growth, resembling or identical with the tumorigenic condition caused by an oncogenic agent. Oncogenic agents have the ability to transform cells so that they grow in a manner analogous to tumor cells. Oncogenes are e.g. IGF-1, EGF, HER-2, src, ras, myc and others. Preferred oncogenic agents of the present invention are e.g. IGF-1 and EGF. The biological process replicative senescence encompasses a process in tissue culture during which primary cells cease to divide after several generations and this process is thought to mimic aging. Apoptosis means programmed cell death and is induced by UV radiation, growth factor depletion, oncogenic drugs etc. Drug susceptibility denotes the sensitivity of cells towards a particular drug, such as oncolytic agents, hormones, cytokines etc.

Preferably the CD2 expression is analyzed by a CD2 specific antibody and is detected by flow cytometry. Flow cytometry was carried out according to protocols known to the person skilled in the art.

In another preferred embodiment the susceptible cells of step a) allow a controlled and reversible switching from a normal to a transformed state and preferably are resistant to G418. An example of such cells is a cell line derived from NIH3T3 fibroblasts after transducing a tetracycline sensitive (tet-off system) human IGF-1 receptor (IGF-IR) gene and selecting for clones with a tight anhydrotetracycline (ATC) repressible promoter (Baasner, S. et al. (1996) *Oncogene* **13,** 901-11).

In a preferred embodiment the method is used for drug-target discovery as secreted and membrane-bound proteins are easily accessible to specific agonists or antagonists.

The present invention is further directed to a non-human embryonic stem cell or non-human transgenic animal comprising the plasmid or vector according to the invention, integrated into its genome. The transgenic animal can be for example a mouse or a rat.

The term "in-frame" denotes a fusion such that the original open reading frame of the sequences is conserved.

The term "retrovirus" refers to any RNA virus that replicates through a DNA intermediate. Such viruses can include those that require the presence of other viruses, such as helper viruses, to be passaged. Thus retroviruses are intended to include those containing substantial deletions or mutations in their RNA. Furthermore, the term "provirus" denotes a viral genome which has integrated into the chromosomal DNA of a cell.

The term "integrated sequence" refers to any nucleic acid sequence, which when contacted with genomic DNA under appropriate conditions, causes the nucleic acid sequence or portion thereof to fuse with the genomic DNA and disrupt the expression of a gene under appropriate conditions. Such integrations - particularly proviruses - cause little if any damage to the adjacent DNA except for interrupting the genomic sequence. Integrated sequences may be included in circularized nucleic acids or in linear nucleic acids, in plasmids or in retroviruses.

The term "analyzing protein expression" denotes any test or series of tests that permits cells expressing the protein to be distinguished from those that do not express the protein. Such tests include biochemical and biological tests.

The term "cell" as used herein encompasses any eukaryotic cell. The cell may be a unicellular organism, part of a multicellular organism, or a fused or engineered cell in culture. The cell may also be a part of an animal, and in one aspect of the invention is part of a transgenic animal, preferably a mammal, most preferably a mouse.

The term "vector" designates an agent (plasmid or virus) used to transmit genetic material to a cell or organism.

The term "susceptible cell" is directed to cells that can be transduced by an expression vector, be it by transfection (plasmid) or infection (retrovirus).

The term "reporter gene" means herein a promoterless coding unit whose product is easily assayed; it may be used to assay function. Examples of reporter genes include neo, geo, hygro, and puro, whereas in the invention neomycin-phosphotransferase (neo) is preferably used.

The term "signal sequence" specifies a short peptide that determines the eventual location of a protein in the cell. An example is the N-terminal sequence of about 20 amino acids that directs nascent secretory and transmembrane proteins to the endoplasmic reticulum.

The term "CD2-positive library" as used herein denotes a set of cells which expresses the CD2 fusion protein of the invention.

The term "CD2-negative library" as used herein designates a set of cells which does not express the CD2 fusion protein of the invention.

The plasmid or retroviral vector according to the invention can be used for effectively selecting for integrations into genes encoding secreted and/or transmembrane proteins that are repressed by oncogenic transformation. This enables a genome-wide screen for proteins of high biological significance, e.g. tumor suppressors that are easily accessible to drugs.

The method of the present invention provides a gene trap strategy enabling genome-wide screening for putative biological process regulator genes, e.g. tumor suppressor genes encoding secreted and/or cell surface proteins in living cells. By infecting a conditionally transformable cell line with the plasmid or retroviral vector according to the invention and selecting in G418, recombinants were obtained that expressed a functional CD2/reporter gene fusion protein on the cell surface. The plasmid or vector of the present invention is advantageous since the signal peptide sequence in the CD2 cDNA terminates in a cryptic splice acceptor site (Andreu, T., et al. (1998) *J Biol Chem* **273**, 13848-54), and it is removed by splicing due to integrations into introns of expressed genes. As a consequence, the fusion gene's activation relies on the acquisition of a signal sequence from an endogenous gene. In each case, transport to the cell membrane was enabled by the acquisition of an N-terminal signal peptide encoded by an endogenous gene. Thus, by using the a plasmid or retroviral vector according to the invention for each reading frame, it is possible to tag all secreted and transmembrane proteins expressed in the mammalian genome. The plasmid or retroviral vector according to the invention with its signal sequence capture frequency of over 86%, is ideally suited for large scale functional genomics.

The plasmid or vector according to the invention offers the ability to select for a subset of genes that are not only encoding secreted and/or cell surface proteins but are also regulated during a biological process. In particular, the plasmid or vector of the present invention enables the recovery of genes involved in oncogenesis and tumor suppression. This could be demonstrated by selecting for and against the plasmid or vector expression in a reversible oncogenic transformation model (see Example 7).

Genes disrupted by the plasmid or vector according to the invention and repressed by oncogenic transformation include: receptor-like protein tyrosine phosphatase κ, thrombin receptor, platelet derived growth factor A chain, α-2 type I collagen gene, Ly-6A.2 alloantigen and class I major histocompatibility complex region Q (see table 2). Indeed, each of the genes disrupted by the plasmid or vector according to the invention had been previously shown to interfere with tumor growth and metastatic spread. The human homologue of the thrombin receptor (PAR-1) and the PDGF A-chain homodimer induce cell cycle arrest and apoptosis by upregulating cyclindependent kinase inhibitors and caspases (Huang, Y. Q., et al. (2000) *J Biol Chem* **275,** 6462-8, Yu, J., et al. (2000) *J Biol Chem* **275,** 19076-82). The α -2 type 1 collagen and the Ly-6A.2 alloantigen inhibit cellular transformation and proliferation (Travers, H., et al. (1996) *Cell Growth & Diff*. **7*,*** 1353-1360, Satoh, M., et al. (1997) *Exp Hematol* **25,** 972-9). Moreover, Ly-6A.2, also known as stem cell antigen-1 (Sca-1) is expressed by primitive stem cells of the hematopoietic system and mediates cell adhesion (English, A., et al. (2000) *J Immunol* **165,** 3763-71). Similarly, cell adhesion is promoted by the membrane bound receptor-like protein tyrosine phosphatase κ (R-PTP- κ, suggesting that both proteins are likely to reduce metastatic spread (Fuchs, M., et al. (1996) *J Biol Chem* **271,** 16712-9). Finally, the class I major histocompatibility complex (MHC-1) is essential for the recognition of tumor antigens by the immune system. In many cancers, downregulation of MHC-1 by tumor cells enables them to evade a specific immune response (Seliger, B., et al. (2000) *Immunol Today* **21,** 455-64).

In conclusion, the methods of the present invention provide a means to carry out genome-wide screens for secreted and/or transmembrane proteins regulated during a specific biological process. Although reversible transformation systems such as the one used in the present invention are particularly attractive for drug-target discovery in cancer research, the approach is adaptable to almost any other biological system where altered signal transduction leads to a detectable phenotype, e.g. cell differentiation, senescence, apoptosis or drug susceptibility. Particularly interesting in this regard are the postgenomic large scale mouse mutagenesis programs which are certain to benefit from the unique features of the plasmid or vector according to the invention (Wiles, M. V., et al. (2000) *Nat Genet* **24,** 13-4).

### DESCRIPTION OF FIGURES

The present invention is further illustrated by the following figures:
**Figure 1: A.** The complete cDNA sequence of CD2 cell surface antigen (SEQ ID NO:1), **B.** Nt 10-782 after the translation start site of the CD2 cell surface antigen cDNA (SEQ ID NO:4)

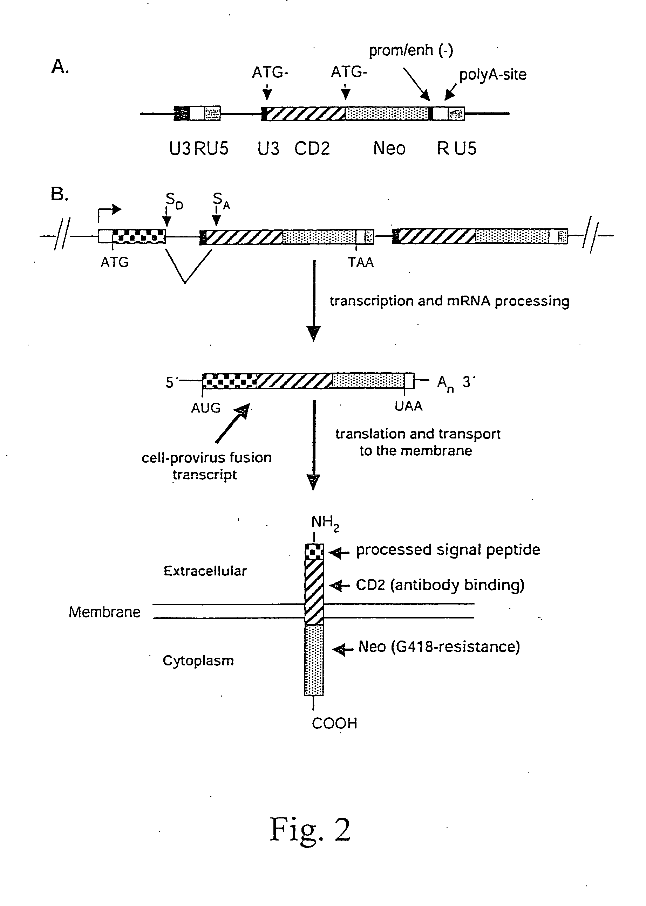

**Figure 2:** Mechanism of signal sequence capture by the plasmid or vector of the invention. **A**. A vector construct (U3Ceo) containing a CD2/neomycin-phosphotransferase fusion gene (Ceo) in U3 region of the 3'LTR, prom/enh (-) = promoter/enhancer deleted. **B.** Mechanism of U3Ceo activation. LTR mediated duplication places the Ceo fusion gene 30 nucleotides from the 5' chromosomal flanking regions. Proviral integrations into the introns of expressed genes result in splicing of upstream exons to the Ceo fusion gene downstream of its cryptic splice acceptor site. In-frame acquisition of a signal sequence from an endogenous gene enables Ceo transport to the cell membrane and converts cells to CD2 positivity and G418-resistance.

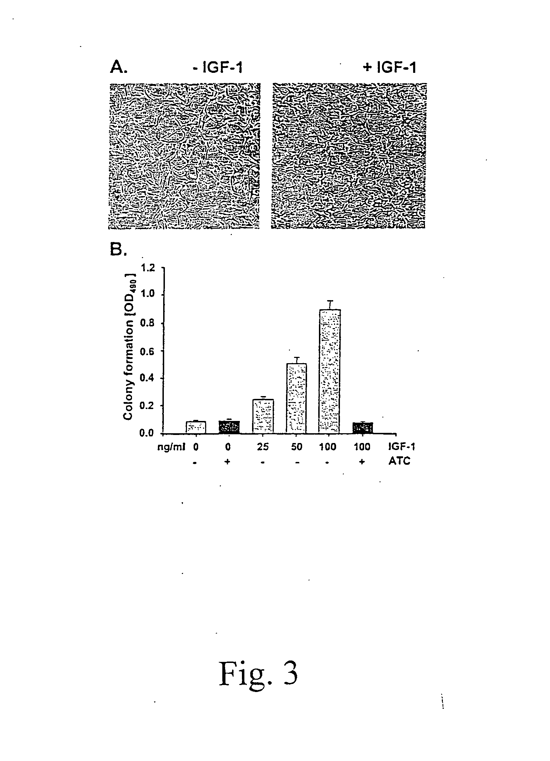

**Figure 3:** IGF-1 induced transformation of N93.1/28 cells. **A.** Focus formation of IGF-1R overexpressing of N93.1/28 cells (- ATC) in presence of IGF-1. **B.** Colony formation in soft agar. 1.5 x 10³ N93.1/28 cells were incubated for 5 days in semisolid cultures ± IGF-1 or anhydrotetracycline (ATC). Colony growth was quantified by measuring light absorption at 490 nm using a spectrophotometer as described in the Example 2.

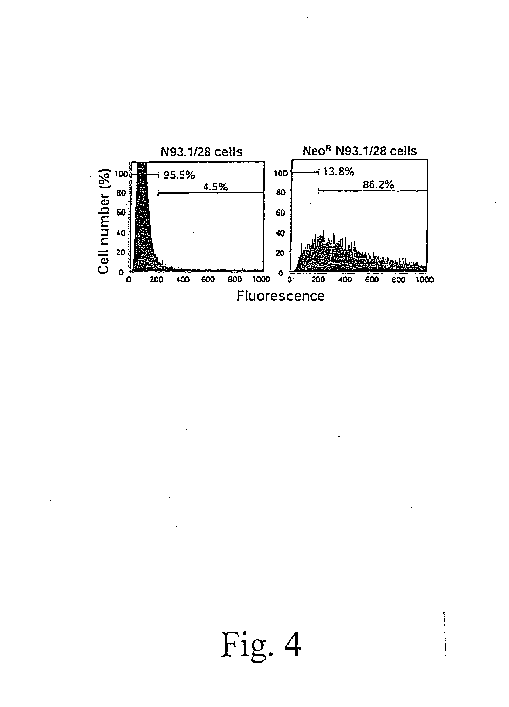

**Figure 4:** CD2 expression in G418-resistant (Neo^{R}) N93.1/28 cells. 1x10⁶ cells were infected with U3Ceo retrovirus at a MOI = 1 and selected in G418 (800µg/ml). 2 x 10⁴ Neo^{R} cells were treated with the monoclonal mouse anti-human CD2 antibody Leu5b. CD2 expression was estimated by flow cytometry after treating the cells with a FITC-conjugated anti-mouse IgG antibody.

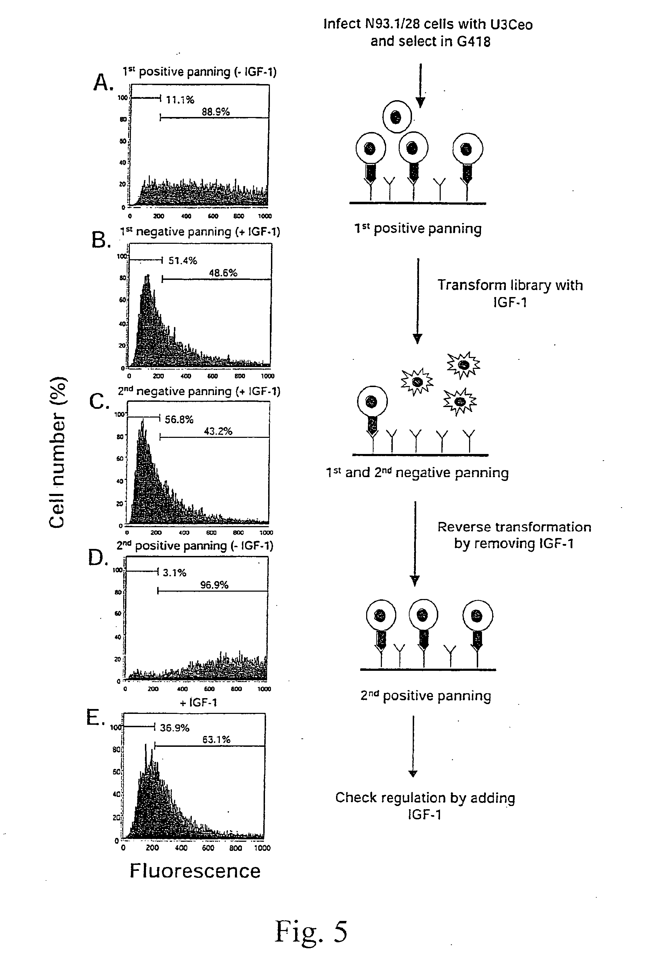

**Figure 5:** Sequential enrichment for U3Ceo integrations repressed by transformation. A U3Ceo integration library consisting of approximately 1 x 10⁷ N93.1/28 cells were subjected to several rounds of "panning" after selecting in G418. Following selection for CD2 expression (positive panning), the library was transformed by IGF-1 and selected against CD2 expression (negative panning). Stepwise enrichment for U3Ceo integrations into IGF-1 regulated genes was estimated by flow cytometry as described in the Legend to Figure 4. A = Fluorescence profile after positive panning of untreated cells; B, C = Fluorescence profile after two consecutive rounds of negative panning of IGF-1 transformed cells; D, E = Fluorescence profile of the preselected library after a second round of positive panning ± IGF-1.

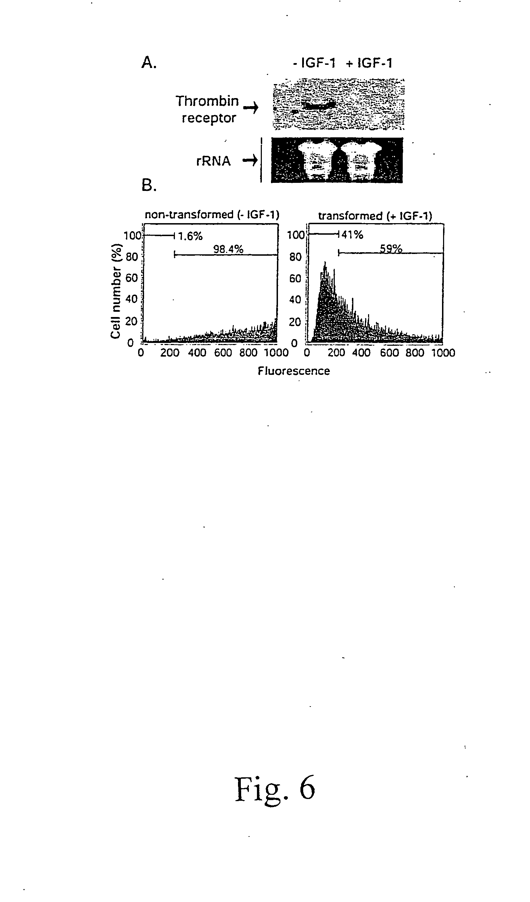

**Figure 6:** IGF-1 regulated expression of the thrombin receptor gene disrupted by U3Ceo in N93.1/28 cells. **A.** Northern blot analysis of the thrombin receptor transcript expressed from the undisrupted allele. N93.1/28 cells were incubated for 72 hours with or without 100ng/ml IGF-1. Total RNAs (25 µg/lane) were fractionated on formaldehyde-agarose gels, blotted onto nylon filters and hybridized to a ³²P labeled thrombin receptor-specific probe. Hybridizing transcripts were visualized by a phosphorimager after exposing for 5 hours to a phosphorimager screen. **B**. Analysis of U3Ceo expression from the allele disrupted by the provirus. IGF-1 regulated CD2 expression was analyzed by flow cytometry as described in the Legend to Figure 4.

### EXAMPLES

The invention is exemplified by the following illustrative but non-limiting examples:

### Example 1: Plasmids and viruses; Construction of a gene trap retrovirus expressing a CD2/neomycin- phosphotransferase fusion gene (U3Ceo)

To construct a gene trap which would allow selection for integrations into genes with signal sequences, the combined reporter/selectable marker CD2/neomycin-phosphotransferase fusion gene (Ceo) was cloned into the U3-region of a promoter/enhancer-deleted retroviral vector based on pBabePuro (Morgenstern, et al. (1990) *Nucleic Acids Res* **18,** 3587-96). Ceo was inserted into the Nhel cloning site of a pBabeSin retroviral vector to obtain pBabeU3Ceo. pBabeSin was derived from pBabePuro (Morgenstern, et al. (1990) *Nucleic Acids Res* **18**, 3587-96) by removing SV40puro and the U3 promoter/enhancer sequences from the 3'LTR. To this end, pBabePuro was cleaved Sall/Xhol and Nhel/Banll, respectively, and religated. High-titer U3Ceo retroviral supernatants were generated by transient transfection of pBabeU3Ceo into BOSC23 packaging cells and used for infections as previously described (Russ, A. P et al. (1996) *J Virol* **70,** 4927-4932).

The Ceo gene was an in-frame fusion between the genes encoding the human T-cell-specific CD2 antigen (CD2) (Seed, B. & Aruffo, A. (1987) *Proc Natl Acad Sci USA* **84,** 3365-9) and the E. coli neomycin-phosphotransferase (neo) (Colbere-Garapin, F., et al (1981) *J Mol Biol* **150,** 1-14). It was obtained by fusing a translation start site deleted and truncated CD2 cDNA to the neo gene immediately downstream of its first ATG (Figure 2A). In particular, Ceo was constructed by amplifying CD2 and neomycin-phosphotransferase by PCR using the primer pairs 5'-attctagattccatgtaaatttgtagccagcttcc-3' (SEQ ID NO: 5)/5'-cgcgggggatccgtagctactctgtgggctcttgtctc-3' (SEQ ID NO: 6) and 5'-cgcgggggatcccattgaacaagatggattgcacgcagg-3' (SEQ ID NO: 7)/5'-cgcggggaattctctagattag-aag-aactcgtcaagaaggcg-3' (SEQ ID NO: 8), respectively. The CD2-amplification product was ligated as an Xbal/BamHI fragment to the 5' BamHI site of the amplified neomycin-phosphotransferase sequence and the fusion gene was cloned as a Xbal/EcoRI fragment into pBluescript KS. After verifying the in-frame fusion by sequencing (ABI310 Genetic Analyzer, Applied Biosystems), the fusion gene was ligated as an Xbal fragment into the Nhel site of pBabe Sin.

The truncated CD2 cDNA, which consists of nucleotides 10 - 782 after the translation start site (Figure 1 , SEQ ID NO:4) and which has no translation start site, encodes the extracellular- and transmembrane domains but only a short segment of the intracellular domain. As has been shown in previous studies, virus replication and long terminal repeat (LTR)-mediated duplication places sequences inserted into U3 just 30 nucleotides downstream from the flanking chromosomal DNA (von Melchner, H. & Ruley, H. E. (1989) *J Virol* **63,** 3227-33). Due to a cryptic splice acceptor sequence located in the CD2 coding sequence, 58 nucleotides downstream of the ATG, and a branch site consensus sequence located 21 nucleotides upstream of the splice site (nucleotides 41-47), gene trap integrations into the introns of transcribed genes were expected to express cell-provirus fusion transcripts in which the upstream exons are spliced to CD2. Since this excises CD2's signal sequence, U3Ceo expression is dependent on an in-frame fusion to a signal sequence of a cellular gene (Figure 2B). Selection for U3Ceo expression enriches then for integrations into genes with signal sequences.

### Example 2: Cell cultures and colony assays; Development of a cell line susceptible to reversible oncogenic transformation

To identify genes that are repressed by oncogenic transformation, a susceptible cell system was required that would allow a controlled and reversible switching from a normal to a transformed state. A susceptible cell line with such properties can be derived from NIH3T3 fibroblasts after transducing a tetracycline sensitive (tet-off system) human IGF-1 receptor (IGF-1R) gene and selecting for clones with a tight anhydrotetracycline (ATC) repressible promoter (Baasner, S., et al. (1996) *Oncogene* **13,** 901-11). One cell line which was obtained in this way and used in the invention has been designated N93.1/28. NIH3T3 cells overexpressing the human IGF-1 receptor under control of the tetracycline-regulated promoter were generated by stable transfection as previously described (Baasner, S., et al. (1996) *Oncogene* **13,** 901-11). Clones isolated by limiting dilution were tested for tetracycline regulated IGF1-R expression by Northern blotting and by reversible tumorigenicity in vitro as previously described (Andreu, T., et al., H. (1998) *J Biol Chem* **273,** 13848-54). When N93.1/28 cells were exposed to IGF-1, they converted to anchorage-independent growth and formed foci in standard focus-forming assays (Figure 3). Conversion was dose dependent and could be readily reversed by ATC, indicating that IGF-1R signaling is required for transformation (Figure 3B) (Baserga, R., et al. (1997) *Biochim Biophys Acta* **1332,** 105-26). The cell line N93.1/28 with tightly regulated IGF-1 R expression was selected for all experiments.

Cells were grown in DMEM supplemented with 10% newborn calf serum (NCS). Anchorage independent growth was analyzed in soft agar colony assays by plating 1.5 x 10³ cells into 96-well plates containing 0.5% w/v DMEM/agar supplemented with 10% (v/v) NCS. After incubating for 7 days, cell proliferation was estimated using the XTT-Cell Proliferation Kit (Roche Diagnostics, Mannheim) according to the manufacturer's instructions. Light absorption reflecting colony proliferation was measured at 490 nm using a Victor 1420 multilabel counter (Wallac, Turku).

### Example 3: Panning and antibody treatment; Analyzing the CD2-expression and generating CD2-positive/negative libraries

The positive and negative panning procedures with the human CD2-specific mouse monoclonal antibody Leu-5b (Becton Dickinson, Heidelberg) were performed as follows: 100-mm bacterial plates were treated with 10ml of a 20µg/ml solution of anti-mouse-lgG-antibody (Cappel, Durham). After incubating for 1.5 h, plates were washed three times with 0.15M NaCI and overlaid with 10 ml of 1% (w/v) solution of bovine serum albumin in PBS. After incubating overnight at room temperature, the bovine serum albumin was removed and plates were stored at -20°C until use. To identify genes repressed by IGF-1, 1 x 10⁷ N93.1/28 cells were infected with U3Ceo at MOI = 1 and first selected for 10 days in G418 (800µg/ml). For panning, cells were suspended at a concentration of 5 x 10⁶/ml and incubated for 30 minutes at room temperature in a 300 ng/ml Leu-5b antibody solution. After washing in PBS, the antibody-treated cells were placed on top of the anti-mouse-lgG antibody coated plates. After incubating for 1 hour at room temperature, plates were gently washed with 10 ml PBS to discard the non-adherent cells. Adherent cells were subsequently harvested in DMEM medium supplemented with 10% NCS and exposed to 100ng/ml recombinant human IGF-1 (Sigma, Deisenhofen) for 3 days. Transformed cells were subjected to negative panning essentially as described above except that this time the nonadherent cells were harvested and the adherent cells discarded.

### Example 4: Flow cytometry; Detecting CD2 expression

1 x 10⁶ cells were suspended in 50µl FACS-Wash solution (Becton Dickinson, Heidelberg) and incubated for 30 min at 4°C with 2.5 µg/ml of CD2-specific mouse monoclonal antibody Leu-5b. Following washings, the cells were incubated for 30 minutes at 4°C in 50 µl FACS-Wash solution containing 10% (v/v) FITC-conjugated anti-mouse-IgG antibody (Roche Diagnostics, Mannheim). Finally, 2 x 10⁴ cells were suspended in 500µl FACS-Wash solution and analyzed with a FACSCALIBUR (Becton Dickinson) flow cytometer using FITC-specific settings.

### Example 5: 5'-RACE

5'-RACE was performed on 5µg of total RNA using the FirstChoice RLM-RACE-Kit (Ambion, Austin) according to the manufacturer's instructions. CD2 specific primers were 5'-caagttgatgtcctgacccaag-3' (SEQ ID NO:9) and 5'-ggtttccaaggcattcgtaatctc-3' (SEQ ID NO: 10) (nested). The first amplification was for 35 cycles, at 96°C for 30sec, 60°C for 30sec, and 72°C for 2min, whereas the second (nested) amplification was for 35 cycles at 96°C for 30sec, 62°C for 30sec, and 72°C for 2min. RACE products were cloned into the pGEM-Teasy vector (Promega, Madison) and inserts of at least two independent E. coli clones were sequenced using a T7- specific primer (Promega).

### Example 6: U3Ceo selects for integrations into genes encoding secreted and transmembrane proteins

To test the ability of U3Ceo to capture cellular signal sequences, non-transformed N93.1/28 cells were infected with U3Ceo virus at a simple multiplicity of infection (MOI = 1) and selected in G418. From a library of 1 x 10⁶ independent integrations, 400 G418-resistant (Neo^{R}) clones were obtained, indicating that only 1 in 2500 integrations are transcribed; this frequency is 25 times below the average number of transcribed integrations obtained with other gene traps activated by gene splicing (10). This suggested that only a small subset of genes expressed in N93.1/28 cells are capable of activating U3Ceo, and led to the assumption that this subset represents the signal sequence genes. If this is indeed the case, the G418-resistant clones should also express CD2 as a result of in-frame fusions between an endogenous signal sequence peptide and the Ceo protein. To test this, pooled Neo^{R} clones were treated with the anti-CD2-specific monoclonal antibody Leu5b and analyzed by flow cytometry for CD2 expression. Figure 4 shows that over 86% of the Neo^{R} cells were positive for CD2, indicating that the majority of U3Ceo fusion proteins have captured an endogenous signal peptide. To investigate this further, cell-provirus fusion transcripts from eight randomly selected Neo^{R} clones were amplified by 5'RNA-ligase mediated (RLM) RACE (Maruyama, K. & Sugano, S. (1994) *Gene* **138,** 171-4) and sequenced. In each case cellular sequences were fused to nucleotide 69 in the CD2 cDNA, which is immediately downstream of the cryptic splice acceptor. Splicing of cellular exons to the Ceo fusion gene deleted the first 90 nucleotides of U3Ceo, including the CD2 signal sequence and the cell DNA-provirus junction. Data base analysis of the fusion transcripts revealed that U3Ceo acquired signal sequences from six previously characterized proteins and from an anonymous EST (Table 1). Thus, taken together, the results indicate that U3Ceo effectively selects for integrations into genes encoding secreted and/or membrane spanning proteins.

**Table 1. Summary of secretion proteins captured by U3Ceo**

| Clone | Origin of signal sequence | Length of signal peptide fused to U3Ceo (aa)* | GenBank Accession number | Function | # of clones |
|---|---|---|---|---|---|
| 1 | Tissue inhibitor of metalloproteinase (TIMP2) | 45 | M82858 | Secreted protein | 1 |
| 2 | Homologue to human mannose-binding lectin | 79 | NM_005570 | Transmembrane protein | 1 |
| 3 | Metalloprotease-disintegrin meltrin β | 25 | AF019887 | Transmembrane protease | 1 |
| 4 | Homologue to rat proteoglykan NG2 | 29 | X56541 | Transmembrane protein | 1 |
| 5 | Lectin λ | 39 | U56734 | Transmembrane receptor | 1 |
| 6 | Homologue to human myoferlin (MYOF) | 48 | AF182316 | Membrane-associated protein | 2 |
| 7 | EST with signal sequence: MAVHACGAAAAWGLL SAAIALQWSPLYA | 29 | Al613763 | unknown | 1 |

| | | | | | |
|---|---|---|---|---|---|
| *In each case the cell-provirus fusion transcripts amplified by 5'-RLM-RACE (25) included the transcriptional start site and the translation initiating AUG of the trapped gene. | | | | | |

### Example 7: Selection for and against U3Ceo expression identifies genes repressed by oncogenic transformation

The value of secreted and cell surface proteins that are involved in transformation is presently unmatched. Therefore, it was investigated whether U3Ceo could be used to identify proteins whose expression is repressed in transformed cells. For this, 1x10⁷ N93.1/28 cells were infected with U3Ceo at a MOI = 1 and the cells were selected for the integration library in G418. The G418-resistant library with U3Ceo integrations in expressed genes was then subjected to several rounds of positive and negative selection using antibody mediated cell adhesion (panning) with the Leu-5b anti-CD2 antibody (Seed, B. & Aruffo, A. (1987) *Proc Natl Acad Sci USA* **84,** 3365-9). As shown in Figure 5A, after a first round of positive panning most cells expressed the CD2 cell surface antigen. However, expression levels varied broadly, as one would expect from a polyclonal population with U3Ceo integrations into genes with variable promoter strength. The CD2-positive library was then transformed by IGF-1 and selected against CD2 expression to eliminate all integrations into constitutively expressed genes (Figure 5 B,C). Finally, to insure that only truly regulated genes were recovered, the negatively selected library was reversed to its non-transformed state by withdrawing IGF-1 and again selecting for CD2 expression. As shown in Figure 5D, the CD2 receptor returned to the cell surface in largely variable amounts, indicating that polyclonality was maintained despite multiple rounds of selection. Moreover, CD2 expression was promptly repressed following IGF-1 addition, implying that the recovered U3Ceo integrations are in tightly regulated genes (Figure 5E).

To identify some of the regulated genes trapped by U3Ceo, six clones were isolated from the preselected library by limiting dilution, and their cell-provirus fusion transcripts were amplified and sequenced. Data base analysis revealed that in each case, U3Ceo had disrupted a gene encoding a signal peptide protein involved in oncogenic transformation and metastatic spread (Table 2). Each of these genes were tightly regulated by IGF-1, as exemplified by the thrombin receptor in Figure 6.

**Table 2. Summary of secretion proteins repressed by oncogenic transformation**

| Clone | Origin of signal sequence | Length of signal peptide fused to U3Ceo (aa)* | GenBank Accession number | Function | Refs. |
|---|---|---|---|---|---|
| 1 | receptor-like protein tyrosine phosphatase κ | 33 | NM_008983 | promotes cell adhesion | Fuchs, M., et al. (1996) *J Biol Chem* **271,** 16712-9. |
| 2 | thrombin receptor | 29 | L03529 | Induces cell-cycle arrest and apop-tosis | Huang, Y. Q., et al. (2000) *J Biol Chem* **275**, 6462-8. |
| 3 | platelet derived growth factor A chain | 54 | NM_008808 | induces apoptosis | Yu, J., et al. (2000) *J Biol Chem* **275**, 19076- 82. |
| 4 | α-2 type I collagen gene | 23 | X58251 | represses ras-mediated transformation | Travers, H., et al. (1996) *Cell Growth* & *Diff.* **7**, 1353- 1360. |
| 5 | Ly-6A.2 alloantigen | 23 | M7352 | Hematopoietic stem cell antigen (Sca-1); promotes cell-cell adhesion and inhibits cell proliferation | Satoh, M., et al. (1997) *Exp Hematol* **25**, 972- 9, English, A., et al. (2000) *J Immunol* **165**, 3763-71. |
| 6 | Class I major histocompatibility complex region Q | 29 | AF111103 | repressed in cancer cells enabling escape from immuno- surveillance | Seliger, B., et al. (2000) *Immunol Today* **21**, 455- 64. |

| | | | | | |
|---|---|---|---|---|---|
| *In each case the cell-provirus fusion transcripts amplified by 5'-RLM-RACE (Maruyama, K. & Sugano, S. (1994) Gene **138**, 171-4) included the transcriptional start site and the translation initiating AUG of the trapped gene. | | | | | |

## Claims

1. A plasmid or a retroviral vector comprising a fusion gene, wherein said fusion gene comprises a nucleic acid sequence encoding the cell surface antigen CD2, wherein said CD2 cell surface antigen has no translation start site and encodes at least the transmembrane and extracellular domains of the CD2 cell surface antigen, fused in frame to a nucleic acid sequence encoding a reporter gene.

2. The plasmid or vector of claim 1, wherein the nucleic acid sequence encodes a truncated CD2 cell surface antigen having the extracellular and transmembrane domains of the CD2 cell surface antigen.

3. The plasmid or vector of claim 1 or 2, wherein said fusion gene comprises the nucleic acid sequence set forth in SEQ ID NO: 3 and the nucleic acid sequence set forth in SEQ ID NO: 2, fused in frame to a nucleic acid sequence encoding a reporter gene.

4. The plasmid or vector of claims 1-3, wherein the nucleic acid sequence encoding a truncated CD2 cell surface antigen comprises the nucleotides 10-782 after the translation start site of the CD2 cDNA.

5. The plasmid or vector of claims 1-4, wherein the fusion gene is inserted in the U3 or U5 region of retroviral vector.

6. The plasmid or vector of any of claims 1-5, wherein the reporter gene is neomycin-phosphotransferase.

7. A method of screening for a nucleic acid sequence encoding a protein comprising a signal sequence comprising the steps of:
(a) transfecting or infecting susceptible cells with a plasmid or vector selected from the group consisting of:
a plasmid or retroviral vector comprising a fusion gene, wherein said fusion gene comprises a nucleic acid sequence encoding the cell surface antigen CD2 having no translation start site and being fused in frame to a nucleic acid encoding a reporter gene;
a plasmid or retroviral vector comprising a fusion gene, wherein said fusion gene comprises a nucleic acid sequence encoding a truncated cell surface antigen CD2 having no translation start site and being fused in frame to a nucleic acid encoding a reporter gene; and the plasmid or vector of any one of claims 1-6,
(b) selecting the cells wherein the plasmid or vector had integrated into the genome, wherein the CD2 signal sequence had been excised by splicing and wherein the integrated plasmid or vector is transcribed,
(c) detecting expression of CD2 or of the fused reporter gene in the selected cells, wherein expression of CD2 or of the fused reporter gene is indicative of an integration of the plasmid or vector into a nucleic acid sequenc3e encoding a protein comprising a signal sequence, and
(d) isolating said CD2-positive and/or fused reporter gene expressing cells.

8. The method of claim 7, wherein the nucleic acid sequence encodes a protein comprising a signal sequence, wherein said protein is regulated during a biological process, further comprising the steps of:
e) generating a CD2-positive library of CD2 expressing cells,
f) treating the CD2-positive library with an agent, which initiates a biological process,
g) analyzing the CD2 expression of the cells, wherein lack of CD2 expression is indicative of integration of the plasmid or vector into a nucleic acid sequence encoding a protein with a signal sequence regulated in a biological process,
h) selecting the cells which do not express CD2,
i) generating a CD2-negative library,
j) withdrawing said agent, thereby terminating said biological process,
k) analyzing the CD2 expression of the cells, wherein the induction of CD2 expression is indicative of an integration of the plasmid or vector into nucleic acid sequence encoding a protein with a signal sequence, wherein said protein is regulated during the biological process, and
I) isolating the CD-2 positive cells.

9. The method of claim 7 or 8 further comprising the step of
m) amplifying cellular sequences adjacent to the plasmid or provirus by genomic PCR and/or amplifying cell-plasmid/provirus fusion transcripts by RT-PCR,
n) sequencing amplification products, and
o) gene identification by data base searches with the sequence of the amplification products.

10. The method of any one of claims 7-9, wherein said signal sequence is a signal sequence of a secreted or of a transmembrane proteins.

11. The method of any one of claims 8-10, wherein said biological process is oncogenic transformation, cell differentiation, senescence, apoptosis or drug susceptibility.

12. The method of any of claims 8-11, wherein the agent of steps f) and j) is a hormone, cytokine, growth factor, oncogene or drug.

13. The method of any of claims 8-11, wherein the agent of steps f) and j) is IGF-1.

14. The method of any one of claims 7-12, wherein the CD2 expression is analyzed by a CD2 specific antibody.

15. The method of any one of claims 7-13, wherein the CD2 expression is detected by flow cytometry.

16. The method of any one of claims 7 to 14 wherein the susceptible cells of step
a) allow a controlled and reversible switching from a normal to a transformed state.

17. The method of claim 15 wherein the susceptible cells are resistent for G418.

18. A non-human embryonic stem cell comprising a vector selected from the group consisting of:
a retroviral vector comprising a fusion gene, wherein said fusion gene comprises a nucleic acid sequence encoding the cell surface antigen CD2 having no translation start site and being fused in frame to a nucleic acid encoding a reporter gene;
a retroviral vector comprising a fusion gene, wherein said fusion gene comprises a nucleic acid sequence encoding truncated cell surface antigen CD2 having no translation start site and being fused in frame to a nucleic acid encoding a reporter gene; and
the vector of any one of claims 1-6,
integrated into its genome.

19. A non-human transgenic animal comprising a plasmid or vector selected from the group consisting of:
a plasmid or retroviral vector comprising a fusion gene, wherein said fusion gene comprises a nucleic acid sequence encoding the cell surface antigen CD2 having no translation start site and being fused in frame to a nucleic acid encoding a reporter gene;
a plasmid or retroviral vector comprising a fusion gene, wherein said fusion gene comprises a nucleic acid sequence encoding a truncated cell surface antigen CD2 having no translation start site and being fused in frame to a nucleic acid encoding a reporter gene; and
the plasmid or vector of any one of claims 1-6,
integrated into its genome.

20. Use of the method of any of claims 7-17 for drug-target discovery.

## Patentansprüche

1. Plasmid oder retroviraler Vektor, das bzw. der ein Fusionsgen umfasst, wobei das Fusionsgen eine Nucleinsäuresequenz, die das Zelloberflächenantigen CD2 codiert, wobei das CD2-Zelloberflächenantigen-Gen keine Translationsstartstelle aufweist und wenigstens die Transmembran- und die extrazelluläre Domäne des CD2-Zelloberflächenantigens codiert, umfasst, die in demselben Raster mit einer Nucleinsäuresequenz, die ein Reportergen codiert, fusioniert ist.

2. Plasmid oder Vektor gemäß Anspruch 1, wobei die Nucleinsäuresequenz ein verkürztes CD2-Zelloberflächenantigen codiert, das die extrazelluläre und die Transmembrandomäne des CD2-Zelloberflächenantigens aufweist.

3. Plasmid oder Vektor gemäß Anspruch 1 oder 2, wobei das Fusionsgen die in SEQ ID Nr. 3 gezeigte Nucleinsäuresequenz und die in SEQ ID Nr. 2 gezeigte Nucleinsäuresequenz umfasst, die in demselben Raster mit einer Nucleinsäuresequenz fusioniert sind, die ein Reportergen codiert.

4. Plasmid oder Vektor gemäß den Ansprüchen 1 bis 3, wobei die Nucleinsäuresequenz, die ein verkürztes CD2-Zelloberflächenantigen codiert, die Nucleotide 10-782 nach der Translationsstartstelle der CD2-cDNA umfasst.

5. Plasmid oder Vektor gemäß den Ansprüchen 1 bis 4, wobei das Fusionsgen in den U3- oder U5-Bereich des retroviralen Vektors eingefügt ist.

6. Plasmid oder Vektor gemäß einem der Ansprüche 1 bis 5, wobei das Reportergen ein Gen für Neomycin-Phosphotransferase ist.

7. Verfahren zum Suchen nach einer Nucleinsäuresequenz, die ein Protein codiert, das eine Signalsequenz umfasst, die folgenden Schritte umfassend:
(a) Transfizieren oder Infizieren von dafür anfälligen Zellen mit einem Plasmid oder Vektor, das bzw. der aus der Gruppe ausgewählt ist, die aus Folgendem besteht:
einem Plasmid oder retroviralen Vektor, das bzw. der ein Fusionsgen umfasst, wobei das Fusionsgen eine Nucleinsäuresequenz umfasst, die das Zelloberflächenantigen CD2 codiert und keine Translationsstartstelle aufweist und in demselben Raster mit einer Nucleinsäuresequenz, die ein Reportergen codiert, fusioniert ist;
einem Plasmid oder retroviralen Vektor, das bzw. der ein Fusionsgen umfasst, wobei das Fusionsgen eine Nucleinsäuresequenz umfasst, die ein verkürztes CD2-Zelloberflächenantigen codiert und keine Translationsstartstelle aufweist und in demselben Raster mit einer Nucleinsäuresequenz, die ein Reportergen codiert, fusioniert ist; und
dem Plasmid oder Vektor gemäß einem der Ansprüche 1 bis 6;
(b) Auswählen der Zellen, wobei das Plasmid bzw. der Vektor in das Genom integriert wurde, wobei die CD2-Signalsequenz durch Spleißen herausgeschnitten wurde und wobei das integrierte Plasmid bzw. der integrierte Vektor transcribiert wird;
(c) Nachweisen der Expression von CD2 oder des fusionierten Reporter-Gens in den ausgewählten Zellen, wobei die Expression von CD2 oder des fusionierten Reporter-Gens eine Integration des Plasmids oder Vektors in eine Nucleinsäuresequenz, die ein Protein codiert, das eine Signalsequenz umfasst, anzeigt; und
(d) Isolieren der CD-2-positiven und/oder das fusionierte Reportergen exprimierenden Zellen.

8. Verfahren gemäß Anspruch 7, wobei die Nucleinsäuresequenz ein Protein codiert, das eine Signalsequenz umfasst, wobei das Protein während eines biologischen Vorgangs reguliert wird, weiterhin die folgenden Schritte umfassend:
e) Erzeugen einer CD2-positiven Bibliothek von CD2-exprimierenden Zellen;
f) Behandeln der CD2-positiven Bibliothek mit einem Mittel, das einen biologischen Vorgang einleitet;
g) Analysieren der CD2-Expression der Zellen, wobei eine fehlende CD2-Expression die Integration des Plasmids oder Vektors in eine Nucleinsäuresequenz, die ein Protein mit einer in einem biologischen Vorgang regulierten Signalsequenz codiert, anzeigt;
h) Selektieren der Zellen, die kein CD2 exprimieren;
i) Erzeugen einer CD2-negativen Bibliothek;
j) Entziehen des Mittels, wodurch der biologische Vorgang beendet wird;
k) Analysieren der CD2-Expression der Zellen, wobei die Induktion der CD2-Expression die Integration des Plasmids oder Vektors in eine Nucleinsäuresequenz, die ein Protein mit einer Signalsequenz codiert, anzeigt, wobei das Protein während des biologischen Vorgangs reguliert wird; und
I) Isolieren der CD-2-positiven Zellen.

9. Verfahren gemäß Anspruch 7 oder 8, das weiterhin die folgenden Schritte umfasst:
m) Amplifizieren von zellulären Sequenzen, die dem Plasmid oder Provirus benachbart sind, durch genomische PCR und/oder Amplifizieren von Zell-Plasmid/Provirus-Fusionstranscripten durch RT-PCR;
n) Sequenzieren der Amplifikationsprodukte; und
o) Genidentifizierung durch Datenbankrecherchen mit der Sequenz der Amplifikationsprodukte.

10. Verfahren gemäß einem der Ansprüche 7 bis 9, wobei die Signalsequenz eine Signalsequenz eines sezernierten Proteins oder eines Transmembranproteins ist.

11. Verfahren gemäß einem der Ansprüche 8 bis 10, wobei es sich bei dem biologischen Vorgang um onkogene Transformation, Zelldifferenzierung, Seneszenz, Apoptose oder Wirkstoffanfälligkeit handelt.

12. Verfahren gemäß einem der Ansprüche 8 bis 11, wobei das Mittel der Schritte f) und j) ein Hormon, Cytokin, Wachstumsfaktor, Onkogen oder Wirkstoff ist.

13. Verfahren gemäß einem der Ansprüche 8 bis 11, wobei es sich bei dem Mittel der Schritte f) und j) um IGF-1 handelt.

14. Verfahren gemäß einem der Ansprüche 7 bis 12, wobei die CD2-Expression mit einem CD2-spezifischen Antikörper analysiert wird.

15. Verfahren gemäß einem der Ansprüche 7 bis 13, wobei die CD2-Expression mittels Durchflusscytometrie nachgewiesen wird.

16. Verfahren gemäß einem der Ansprüche 7 bis 14, wobei die anfälligen Zellen von Schritt a) ein gesteuertes und reversibles Umschalten von einem normalen zu einem transformierten Zustand erlauben.

17. Verfahren gemäß Anspruch 15, wobei die anfälligen Zellen gegenüber G418 resistent sind.

18. Nichthumane embryonale Stammzelle, die in ihr Genom integriert einen Vektor umfasst, der aus der Gruppe ausgewählt ist, die aus folgenden besteht:
einem retroviralen Vektor, der ein Fusionsgen umfasst, wobei das Fusionsgen eine Nucleinsäuresequenz umfasst, die das Zelloberflächenantigen CD2 codiert und keine Translationsstartstelle aufweist und in demselben Raster mit einer Nucleinsäure, die ein Reportergen codiert, fusioniert ist;
einem retroviralen Vektor, der ein Fusionsgen umfasst, wobei das Fusionsgen eine Nucleinsäuresequenz umfasst, die ein verkürztes CD2-Zelloberflächenantigen codiert und keine Translationsstartstelle aufweist und in demselben Raster mit einer Nucleinsäuresequenz, die ein Reportergen codiert, fusioniert ist; und
dem Vektor gemäß einem der Ansprüche 1 bis 6.

19. Nichthumanes transgenes Tier, das in sein Genom integriert ein Plasmid oder einen Vektor umfasst, das bzw. der aus der Gruppe ausgewählt ist, die aus Folgendem besteht:
einem Plasmid oder retroviralen Vektor, das bzw. der ein Fusionsgen umfasst, wobei das Fusionsgen eine Nucleinsäuresequenz umfasst, die das Zelloberflächenantigen CD2 codiert und keine Translationsstartstelle aufweist und in demselben Raster mit einer Nucleinsäuresequenz, die ein Reportergen codiert, fusioniert ist;
einem Plasmid oder retroviralen Vektor, das bzw. der ein Fusionsgen umfasst, wobei das Fusionsgen eine Nucleinsäuresequenz umfasst, die ein verkürztes CD2-Zelloberflächenantigen codiert und keine Translationsstartstelle aufweist und in demselben Raster mit einer Nucleinsäuresequenz, die ein Reportergen codiert, fusioniert ist; und
dem Plasmid oder Vektor gemäß einem der Ansprüche 1 bis 6.

20. Verwendung des Verfahrens gemäß einem der Ansprüche 7 bis 17 zur Entdeckung von Wirkstofftargets.

## Revendications

1. Plasmide ou vecteur rétroviral comprenant un gène de fusion, dans lequel ledit gène de fusion comprend une séquence d'acide nucléique codant pour l'antigène de surface cellulaire CD2, dans lequel ledit antigène de surface cellulaire CD2 ne comporte aucun site d'initiation de la traduction, et codant au moins pour les domaines transmembranaire et extracellulaire de l'antigène de surface cellulaire CD2, fusionnée dans le cadre à une séquence d'acide nucléique codant pour un gène rapporteur.

2. Plasmide ou vecteur selon la revendication 1, dans lequel la séquence d'acide nucléique code pour un antigène de surface cellulaire CD2 tronqué comportant les domaines extracellulaire et transmembranaire de l'antigène de surface cellulaire CD2.

3. Plasmide ou vecteur selon la revendication 1 ou 2, dans lequel ledit gène de fusion comprend la séquence d'acide nucléique représentée dans SEQ ID N° : 3 et la séquence d'acide nucléique représentée dans SEQ ID N° : 2, fusionnées dans le cadre à une séquence d'acide nucléique codant pour un gène rapporteur.

4. Plasmide ou vecteur selon les revendications 1 à 3, dans lequel la séquence d'acide nucléique codant pour un antigène de surface cellulaire CD2 tronqué comprend les nucléotides 10-782 après le site d'initiation de la traduction de l'ADNc de CD2.

5. Plasmide ou vecteur selon les revendications 1 à 4, dans lequel le gène de fusion est inséré dans la région U3 ou U5 d'un vecteur rétroviral.

6. Plasmide ou vecteur selon l'une quelconque des revendications 1 à 5, dans lequel le gène rapporteur est la néomycine phosphotransférase.

7. Procédé de criblage pour une séquence d'acide nucléique codant pour une protéine comportant une séquence signal comprenant les étapes de:
(a) transfection ou infection de cellules sensibles avec un plasmide ou un vecteur choisi dans le groupe constitué par:
un plasmide ou un vecteur rétroviral comprenant un gène de fusion, dans lequel ledit gène de fusion comprend une séquence d'acide nucléique codant pour l'antigène de surface cellulaire CD2 ne comportant aucun site d'initiation de la traduction et étant fusionnée dans le cadre à un acide nucléique codant pour un gène rapporteur ;
un plasmide ou un vecteur rétroviral comprenant un gène de fusion, dans lequel ledit gène de fusion comprend une séquence d'acide nucléique codant pour un antigène de surface cellulaire CD2 tronqué ne comportant aucun site d'initiation de la traduction et étant fusionnée dans le cadre à un acide nucléique codant pour un gène rapporteur ; et
le plasmide ou vecteur selon l'une quelconque des revendications 1 à 6,
(b) sélection des cellules dans lesquelles le plasmide ou le vecteur a intégré le génome, dans lesquelles la séquence signal de CD2 a été excisée par épissage et dans lesquelles le plasmide ou vecteur intégré est transcrit,
(c) détection de l'expression de CD2 ou du gène rapporteur fusionné dans les cellules sélectionnées, dans lesquelles l'expression de CD2 ou du gène rapporteur fusionné indique une intégration du plasmide ou du vecteur dans une séquence d'acide nucléique codant pour protéine comprenant une séquence signal, et
(d) isolement desdites cellules positives à CD2 et/ou exprimant un gène rapporteur fusionné.

8. Procédé selon la revendication 7, dans lequel la séquence d'acide nucléique code pour une protéine comprenant une séquence signal, ladite protéine étant régulée pendant un processus biologique, comprenant en outre les étapes de:
(e) création d'une banque positive à CD2 de cellules exprimant CD2,
(f) traitement de la banque positive à CD2 avec un agent qui initie un processus biologique,
(g) analyse de l'expression de CD2 dans les cellules, une absence d'expression de CD2 indiquant l'intégration du plasmide ou du vecteur dans une séquence d'acide nucléique codant pour une protéine avec une séquence signal régulée dans un processus biologique,
(h) sélection des cellules qui n'expriment pas CD2,
(i) création d'une banque négative à CD2,
(j) retrait dudit agent, interrompant ainsi ledit processus biologique,
(k) analyse de l'expression de CD2 dans les cellules, l'induction de l'expression de CD2 indiquant une intégration du plasmide ou du vecteur dans une séquence d'acide nucléique codant pour une protéine avec une séquence signal, ladite protéine étant régulée pendant le processus biologique, et
(l) isolement des cellules positives à CD2.

9. Procédé selon la revendication 7 ou 8 comprenant en outre les étapes de:
(m) amplification des séquences cellulaires adjacentes au plasmide ou au provirus par PCR génomique et/ou amplification des transcrits de fusion cellule-plasmide/provirus par PCR inverse,
(n) séquençage des produits d'amplification, et
(o) identification de gènes par consultation de bases de données avec la séquence des produits d'amplification.

10. Procédé selon l'une quelconque des revendications 7 à 9, dans lequel ladite séquence signal est une séquence signal d'une protéine sécrétée ou transmembranaire.

11. Procédé selon l'une quelconque des revendications 8 à 10, dans lequel ledit processus biologique est une transformation oncogénique, une différenciation cellulaire, une sénescence, une apoptose ou une sensibilité à un médicament.

12. Procédé selon l'une quelconque des revendications 8 à 11, dans lequel l'agent des étapes (f) et (j) est une hormone, une cytokine, un facteur de croissance, un oncogène ou un médicament.

13. Procédé selon l'une quelconque des revendications 8 à 11, dans lequel l'agent des étapes (f) et (j) est l'IGF-1.

14. Procédé selon l'une quelconque des revendications 7 à 12, dans lequel l'expression de CD2 est analysée par un anticorps spécifique de CD2.

15. Procédé selon l'une quelconque des revendications 7 à 13, dans lequel l'expression de CD2 est détectée par cytométrie en flux.

16. Procédé selon l'une quelconque des revendications 7 à 14, dans lequel les cellules sensibles de l'étape (a) permettent un passage contrôlé et réversible d'un état normal à un état transformé.

17. Procédé selon la revendication 15 dans lequel les cellules sensibles sont résistantes au G418.

18. Cellule souche embryonnaire non humaine comprenant un vecteur choisi dans le groupe constitué par:
un vecteur rétroviral comprenant un gène de fusion, dans lequel ledit gène de fusion comprend une séquence d'acide nucléique codant pour l'antigène de surface cellulaire CD2 ne comportant aucun site d'initiation de la traduction et étant fusionnée dans le cadre à un acide nucléique codant pour un gène rapporteur;
un vecteur rétroviral comprenant un gène de fusion, dans lequel ledit gène de fusion comprend une séquence d'acide nucléique codant pour un antigène de surface cellulaire CD2 tronqué ne comportant aucun site d'initiation de la traduction et étant fusionnée dans le cadre à un acide nucléique codant pour un gène rapporteur ; et
le vecteur selon L'une quelconque des revendications 1 à 6,
intégré dans son génome.

19. Animal transgénique non humain comprenant un plasmide ou un vecteur choisi dans le groupe constitué par:
un plasmide ou un vecteur rétroviral comprenant un gène de fusion, dans lequel ledit gène de fusion comprend une séquence d'acide nucléique codant pour l'antigène de surface cellulaire CD2 ne comportant aucun site d'initiation de la traduction et étant fusionnée dans le cadre à un acide nucléique codant pour un gène rapporteur ;
un plasmide ou un vecteur rétroviral comprenant un gène de fusion, dans lequel ledit gène de fusion comprend une séquence d'acide nucléique codant pour un antigène de surface cellulaire CD2 tronqué ne comportant aucun site d'initiation de la traduction et étant fusionnée dans le cadre à un acide nucléique codant pour un gène rapporteur ; et
le plasmide ou vecteur selon l'une quelconque des revendications 1 à 6,
intégré dans son génome.

20. Utilisation du procédé selon l'une quelconque des revendications 7 à 17 pour la découverte d'une cible médicamenteuse.


### SEQUENCE LISTING

<110> FrankGen Biotechnologie AG
<120> METHODS FOR SCREENING FOR PROTEINS COMPRISING A SIGNAL SEQUENCE
<130> 292-5
<140>
   <141> 2001-07-27
<160> 10
<170> Patent In Ver. 2.1
<210> 1
   <211> 1504
   <212> DNA
   <213> Homo sapiens
<220>
   <223> Human CD2 cell surface antigen cDNA
<400> 1
   cctaagatga gctttccatg taaatttgta gccagcttcc ttctgatttt caatgtttct 60
   tccaaaggtg cagtctccaa agagattacg aatgccttgg aaacctgggg tgccttgggt 120
   caggacatca acttggacat tcctagtttt caaatgagtg atgatattga cgatataaaa 180
   tgggaaaaaa cttcagacaa gaaaaagatt gcacaattca gaaaagagaa agagactttc 240
   aaggaaaaag atacatataa gctatttaaa aatggaactc tgaaaattaa gcatctgaag 300
   accgatgatc aggatatcta caaggtatca atatatgata caaaaggaaa aaatgtgttg 360
   gaaaaaatat ttgatttgaa gattcaagag agggtctcaa aaccaaagat ctcctggact 420
   tgtatcaaca caaccctgac ctgtgaggta atgaatggaa ctgaccccga attaaacctg 480
   tatcaagatg ggaaacatct aaaactttct cagagggtca tcacacacaa gtggaccacc 540
   agcctgagtg caaaattcaa gtgcacagca gggaacaaag tcagcaagga atccagtgtc 600
   gagcctgtca gctgtccaga gaaaggtctg gacatctatc tcatcattgg catatgtgga 660
   ggaggcagcc tcttgatggt ctttgtggca ctgctcgttt tctatatcac caaaaggaaa 720
   aaacagagga gtcggagaaa tgatgaggag ctggagacaa gagcccacag agtagctact 780
   gaagaaaggg gccggaagcc ccaccaaatt ccagcttcaa cccctcagaa tccagcaact 840
   tcccaacatc ctcctccacc acctggtcat cgttcccagg cacctagtca tcgtcccccg 900
   cctcctggac accgtgttca gcaccagcct cagaagaggc ctcctgctcc gtcgggcaca 960
   caagttcacc agcagaaagg cccgcccctc cccagacctc gagttcagcc aaaacctccc 1020
   catggggcag cagaaaactc attgtcccct tcctctaatt aaaaaagata gaaactgtct 1080
   ttttcaataa aaagcactgt ggatttctgc cctcctgatg tgcatatccg tacttccatg 1140
   aggtgttttc tgtgtgcaga acattgtcac ctcctgaggc tgtgggccac agccacctct 1200
   gcatcttcga actcagccat gtggtcaaca tctggagttt ttggtctcct cagagagctc 1260
   catcacacca gtaaggagaa gcaatataag tgtgattgca agaatggtag aggaccgagc 1320
   acagaaatct tagagatttc ttgtcccctc tcaggtcatg tgtagatgcg ataaatcaag 1380
   tgattggtgt gcctgggtct cactacaagc agcctatctg cttaagagac tctggagttt 1440
   cttatgtgcc ctggtggaca cttgcccacc atcctgtgag taaaagtgaa ataaaagctt 1500
   tgac 1504
<210> 2
   <211> 70
   <212> DNA
   <213> Homo sapiens
   <220>
   <223> Nt. 10-79 after the translation start site of CD2 cDNA
<400> 2
   ccatgtaaat ttgtagccag cttccttctg attttcaatg tttcttccaa aggtgcagtc 60
   tccaaagaga 70
<210> 3
   <211> 545
   <212> DNA
   <213> Homo sapiens
<220>
   <223> Nt. 80-624 after the translation start site of CD2 cDNA
<400> 3
   ttacgaatgc cttggaaacc tggggtgcct tgggtcagga catcaacttg gacattccta 60
   gttttcaaat gagtgatgat attgacgata taaaatggga aaaaacttca gacaagaaaa 120
   agattgcaca attcagaaaa gagaaagaga ctttcaagga aaaagataca tataagctat 180
   ttaaaaatgg aactctgaaa attaagcatc tgaagaccga tgatcaggat atctacaagg 240
   tatcaatata tgatacaaaa ggaaaaaatg tgttggaaaa aatatttgat ttgaagattc 300
   aagagagggt ctcaaaacca aagatctcct ggacttgtat caacacaacc ctgacctgtg 360
   aggtaatgaa tggaactgac cccgaattaa acctgtatca agatgggaaa catctaaaac 420
   tttctcagag ggtcatcaca cacaagtgga ccaccagcct gagtgcaaaa ttcaagtgca 480
   cagcagggaa caaagtcagc aaggaatcca gtgtcgagcc tgtcagctgt ccagagaaag 540
   gtctg                                                             545
<210> 4
   <211> 773
   <212>DNA
   <213> Homo sapiens
<220>
   <223> Nt. 10-782 after the translation start site of CD2 cDNA
<400> 4
   ccatgtaaat ttgtagccag cttccttctg attttcaatg tttcttccaa aggtgcagtc 60
   tccaaagaga ttacgaatgc cttggaaacc tggggtgcct tgggtcagga catcaacttg 120
   gacattccta gttttcaaat gagtgatgat attgacgata taaaatggga aaaaacttca 180
   gacaagaaaa agattgcaca attcagaaaa gagaaagaga ctttcaagga aaaagataca 240
   tataagctat ttaaaaatgg aactctgaaa attaagcatc tgaagaccga tgatcaggat 300
   atctacaagg tatcaatata tgatacaaaa ggaaaaaatg tgttggaaaa aatatttgat 360
   ttgaagattc aagagagggt ctcaaaacca aagatctcct ggacttgtat caacacaacc 420
   ctgacctgtg aggtaatgaa tggaactgac cccgaattaa acctgtatca agatgggaaa 480
   catctaaaac tttctcagag ggtcatcaca cacaagtgga ccaccagcct gagtgcaaaa 540
   ttcaagtgca cagcagggaa caaagtcagc aaggaatcca gtgtcgagcc tgtcagctgt 600
   ccagagaaag gtctggacat ctatctcatc attggcatat gtggaggagg cagcctcttg 660
   atggtctttg tggcactgct cgttttctat atcaccaaaa ggaaaaaaca gaggagtcgg 720
   agaaatgatg aggagctgga gacaagagcc cacagagtag ctactgaaga aag        773
<210> 5
   <211> 35
   <212> DNA
   <213> Artificial Sequence
<220>
   <223> Description of Artificial Sequence: Primer
<400> 5
   attctagatt ccatgtaaat ttgtagccag cttcc 35
<210> 6
   <211> 38
   <212> DNA
   <213> Artificial Sequence
<220>
   <223> Description of Artificial Sequence: Primer
<400> 6
   cgcgggggat ccgtagctac tctgtgggct cttgtctc 38
<210> 7
   <211> 39
   <212> DNA
   <213> Artificial Sequence
<220>
   <223> Description of Artificial Sequence: Primer
<900> 7
   cgcgggggat cccattgaac aagatggatt gcacgcagg 39
<210> 8
   <211> 42
   <212> DNA
   <213> Artificial Sequence
<220>
   <223> Description of Artificial Sequence: Primer
<400> 8
   cgcggggaat tctctagatt agaagaactc gtcaagaagg cg 42
<210> 9
   <211> 22
   <212> DNA
   <213> Artificial Sequence
<220>
   <223> Description of Artificial Sequence: Primer
<400> 9
   caagttgatg tcctgaccca ag 22
<210> 10
   <211> 24
   <212> DNA
   <213> Artificial Sequence
<220>
   <223> Description of Artificial Sequence: Primer
<400> 10
   ggtttccaag gcattcgtaa tctc 24

### SEQUENCE LISTING

<110> FrankGen Biotechnologie AG
<120> METHODS FOR SCREENING FOR PROTEINS COMPRISING A SIGNAL SEQUENCE
<130> 292-5
<140>
   <141> 2001-07-27
<160> 10
<170> PatentIn Ver. 2.1
<210> 1
   <211> 1504
   <212> DNA
   <213> Homo sapiens
<220>
   <223> Human CD2 cell surface antigen cDNA
<400> 1
   cctaagatga gctttccatg taaatttgta gccagcttcc ttctgatttt caatgtttct 60
   tccaaaggtg cagtctccaa agagattacg aatgccttgg aaacctgggg tgccttgggt 120
   caggacatca acttggacat tcctagtttt caaatgagtg atgatattga cgatataaaa 180
   tgggaaaaaa cttcagacaa gaaaaagatt gcacaattca gaaaagagaa agagactttc 240
   aaggaaaaag atacatataa gctatttaaa aatggaactc tgaaaattaa gcatctgaag 300
   accgatgatc aggatatcta caaggtatca atatatgata caaaaggaaa aaatgtgttg 360
   gaaaaaatat ttgatttgaa gattcaagag agggtctcaa aaccaaagat ctcctggact 420
   tgtatcaaca caaccctgac ctgtgaggta atgaatggaa ctgaccccga attaaacctg 480
   tatcaagatg ggaaacatct aaaactttct cagagggtca tcacacacaa gtggaccacc 540
   agcctgagtg caaaattcaa gtgcacagca gggaacaaag tcagcaagga atccagtgtc 600
   gagcctgtca gctgtccaga gaaaggtctg gacatctatc tcatcattgg catatgtgga 660
   ggaggcagcc tcttgatggt ctttgtggca ctgctcgttt tctatatcac caaaaggaaa 720
   aaacagagga gtcggagaaa tgatgaggag ctggagacaa gagcccacag agtagctact 780
   gaagaaaggg gccggaagcc ccaccaaatt ccagcttcaa cccctcagaa tccagcaact 840
   tcccaacatc ctcctccacc acctggtcat cgttcccagg cacctagtca tcgtcccccg 900
   cctcctggac accgtgttca gcaccagcct cagaagaggc ctcctgctcc gtcgggcaca 960
   caagttcacc agcagaaagg cccgcccctc cccagacctc gagttcagcc aaaacctccc 1020
   catggggcag cagaaaactc attgtcccct tcctctaatt aaaaaagata gaaactgtct 1080
   ttttcaataa aaagcactgt ggatttctgc cctcctgatg tgcatatccg tacttccatg 1140
   aggtgttttc tgtgtgcaga acattgtcac ctcctgaggc tgtgggccac agccacctct 1200
   gcatcttcga actcagccat gtggtcaaca tctggagttt ttggtctcct cagagagctc 1260
   catcacacca gtaaggagaa gcaatataag tgtgattgca agaatggtag aggaccgagc 1320
   acagaaatct tagagatttc ttgtcccctc tcaggtcatg tgtagatgcg ataaatcaag 1380
   tgattggtgt gcctgggtct cactacaagc agcctatctg cttaagagac tctggagttt 1440
   cttatgtgcc ctggtggaca cttgcccacc atcctgtgag taaaagtgaa ataaaagctt 1500
   tgac                                                              1504
<210> 2
   <211> 70
   <212> DNA
   <213> Homo sapiens
   <220>
   <223> Nt. 10-79 after the translation start site of CD2 cDNA
<400> 2
   ccatgtaaat ttgtagccag cttccttctg attttcaatg tttcttccaa aggtgcagtc 60
   tccaaagaga                                                        70
<210> 3
   <211> 545
   <212> DNA
   <213> Homo sapiens
<220>
   <223> Nt. 80-624 after the translation start site of CD2 cDNA
<400> 3
   ttacgaatgc cttggaaacc tggggtgcct tgggtcagga catcaacttg gacattccta 60
   gttttcaaat gagtgatgat attgacgata taaaatggga aaaaacttca gacaagaaaa 120
   agattgcaca attcagaaaa gagaaagaga ctttcaagga aaaagataca tataagctat 180
   ttaaaaatgg aactctgaaa attaagcatc tgaagaccga tgatcaggat atctacaagg 240
   tatcaatata tgatacaaaa ggaaaaaatg tgttggaaaa aatatttgat ttgaagattc 300
   aagagagggt ctcaaaacca aagatctcct ggacttgtat caacacaacc ctgacctgtg 360
   aggtaatgaa tggaactgac cccgaattaa acctgtatca agatgggaaa catctaaaac 420
   tttctcagag ggtcatcaca cacaagtgga ccaccagcct gagtgcaaaa ttcaagtgca 480
   cagcagggaa caaagtcagc aaggaatcca gtgtcgagcc tgtcagctgt ccagagaaag 540
   gtctg                                                             545
<210> 4
   <211> 773
   <212> DNA
   <213> Homo sapiens
<220>
   <223> Nt. 10-782 after the translation start site of CD2 cDNA
<400> 4
   ccatgtaaat ttgtagccag cttccttctg attttcaatg tttcttccaa aggtgcagtc 60
   tccaaagaga ttacgaatgc cttggaaacc tggggtgcct tgggtcagga catcaacttg 120
   gacattccta gttttcaaat gagtgatgat attgacgata taaaatggga aaaaacttca 180
   gacaagaaaa agattgcaca attcagaaaa gagaaagaga ctttcaagga aaaagataca 240
   tataagctat ttaaaaatgg aactctgaaa attaagcatc tgaagaccga tgatcaggat 300
   atctacaagg tatcaatata tgatacaaaa ggaaaaaatg tgttggaaaa aatatttgat 360
   ttgaagattc aagagagggt ctcaaaacca aagatctcct ggacttgtat caacacaacc 420
   ctgacctgtg aggtaatgaa tggaactgac cccgaattaa acctgtatca agatgggaaa 480
   catctaaaac tttctcagag ggtcatcaca cacaagtgga ccaccagcct gagtgcaaaa 540
   ttcaagtgca cagcagggaa caaagtcagc aaggaatcca gtgtcgagcc tgtcagctgt 600
   ccagagaaag gtctggacat ctatctcatc attggcatat gtggaggagg cagcctcttg 660
   atggtctttg tggcactgct cgttttctat atcaccaaaa ggaaaaaaca gaggagtcgg 720
   agaaatgatg aggagctgga gacaagagcc cacagagtag ctactgaaga aag        773
<210> 5
   <211> 35
   <212> DNA
   <213> Artificial Sequence
<220>
   <223> Description of Artificial Sequence: Primer
<400> 5
   attctagatt ccatgtaaat ttgtagccag cttcc 35
<210> 6
   <211> 38
   <212> DNA
   <213> Artificial Sequence
<220>
   <223> Description of Artificial Sequence: Primer
<400> 6
   cgcgggggat ccgtagctac tctgtgggct cttgtctc 38
<210> 7
   <211> 39
   <212> DNA
   <213> Artificial Sequence
<220>
   <223> Description of Artificial Sequence: Primer
<400> 7
   cgcgggggat cccattgaac aagatggatt gcacgcagg 39
<210> 8
   <211> 42
   <212> DNA
   <213> Artificial Sequence
<220>
   <223> Description of Artificial Sequence: Primer
<400> 8
   cgcggggaat tctctagatt agaagaactc gtcaagaagg cg 42
<210> 9
   <211> 22
   <212> DNA
   <213> Artificial Sequence
<220>
   <223> Description of Artificial Sequence: Primer
<400> 9
   caagttgatg tcctgaccca ag 22
<210> 10
   <211> 24
   <212> DNA
   <213> Artificial Sequence
<220>
   <223> Description of Artificial Sequence: Primer
<400> 10
   ggtttccaag gcattcgtaa tctc 24